# EUROPEAN PATENT APPLICATION

(11) **EP 0 814 161 A1**
(43) Date of publication of application: **29.12.1997**
(21) Application number: 96110088.0
(22) Date of filing: 21.06.1996
(51) Int. Cl.: C12N 15/29, C12N 15/82, C07K 14/415, A01H 5/00, C12Q 1/68

(54) **Genetic control of polar auxin transport in plants and manipulation of plant growth, architecture and morphogenesis**

(71) Applicant: Max-Planck-Gesellschaft zur Förderung der Wissenschaften e.V., 80539 München (DE)
(72) Inventor: Gälweiler, Leo, 55595 St. Katharinen (DE); Palme, Klaus, PD Dr., 50259 Pulheim (DE); Wismann, Ellen Dr., 6369 BD Simpelveld (NL); Saedler, Heinz, Prof. Dr., 50829 Köln (DE)
(74) Representative: Maiwald, Walter, Dr. Dipl.-Chem.

(57) **Abstract**

This invention relates to the control of polar auxin transport or auxin activity and of plant hormone levels at the molecular level. It particularly relates to nucleotide sequences of the pin gene, their derivatives and fragments and the encoded gene products involved in polar auxin transport, and a method for the production of new plants carrying at least one of the nucleotide sequences of the invention. Further, the invention relates to new plants showing altered plant growth, architecture, morphogenesis, branching, flowering, fruit production and/or other developmental features affected by auxin.

## Description

This invention relates to the control of polar auxin transport and of plant hormone levels at the molecular level. It particularly relates to nucleotide sequences of the pin gene, their derivatives and fragments and the encoded gene products involved in polar auxin transport, and a method for the production of new plants carrying at least one of the nucleotide sequences of the invention. Further, the invention relates to new plants showing altered plant growth, architecture, morphogenesis, branching, flowering, fruit production and/or other developmental features affected by auxin.

Plant hormones are naturally occuring substances, effective in very small amounts, that act as signals to stimulate or inhibit growth or regulate some other developmental process in higher plants. One of the most common plant hormones is indole-3-ylacetic acid (IAA), which is often referred to as "auxin" and which was the first plant hormone to be identified. The auxin biosynthetic pathway is not known with certainty, but there appears to be one major pathway for the synthesis of IAA in plants via tryptophan, and another one via indole. IAA is found in plants in free form or in the form of ester or amide conjugates.

The definition of a hormone includes the stipulation that the hormone moves through the organism from its site of synthesis to its site of action. Mainly, auxin is synthesized in meristematic and embryonic tissues and juvenile parts of the plant (e.g. young leaves). In dicotyledones the apex, i.e. the tip of the main shoot, is the most important IAA source. Auxin is transported down the stem by a specific mechanism known as the auxin transport system. This energy-dependent polar basipetal (i.e. towards the root) transport, being gravity independent, occurs mainly in vascular tissues. The transport velocity is generally 5-15 mm/h.

Auxin regulates many aspects of plant growth and development. In addition to the regulation of cell elongation, auxin is involved in the control of several other developmental processes throughout the plant. Auxin is well established as the signaling molecule in phototropism. It regulates the elongation of young internodes and the enlargement of the young leaf blades. It also initiates cell differentiation in both leaves and internodes as it stimulates their enlargement or elongation. While promoting the growth of these tissues and organs, it inhibits the development of lateral buds. This leads to a condition known as apical dominance. Auxin inhibits the formation of the abscission zone at the base of the leaves, fruits, and other organs that allows them to drop from the plant. Auxin also regulates vascular tissue differentiation and cell division in the vascular cambium, which of course is involved in secondary growth, and it hase been implicated in the maintenance of tissue polarity.

Although the molecular mechanisms of polar auxin transport are presently not fully understood in detail, it seems likely that specific transport proteins, so-called auxin-efflux-carrier proteins, are involved. Results obtained using chemical compounds which inhibit polar auxin transport, like the phytotropin N-(naphth-1-yl)phthalamic acid (NPA) or 2,3,5-triiodobenzoic acid (TIBA), indicate that such inhibitors compete with IAA for specific auxin receptor proteins, which interact with the carrier proteins, and/or more directly for the specific auxin-carrier proteins (Jacobs and Rubery (1988), Science 241, 346-349; Rubery (1990), Soc. Exp. Biol. Symp. 44, 119-146).

The manipulation of polar auxin transport and the developmental processes affected by auxin is a very important aim of molecular plant breeding, and some efforts have been made to isolate and characterize the genes involved. Several studies focused on plant mutants known to be defective in active auxin transport. For instance, the pin1-1 mutant of Arabidopsis thaliana having a "pin" shape morphology shows several structural abnormalities in different organs at different stages of plant growth. In some cases this mutant forms flowers with abnormal structures at the top of inflorescense axes; in other cases, no floral buds are formed on the axes (Okada et al. (1991), The Plant Cell 3, 677-684). The polar transport activity in the pin1-1 mutant and in the pin1-2 mutant, an independently isolated allelic mutant which shows similar phenotypes as pin1-1, was decreased to 14% and 7% of wild type, respectively (Okada et al., supra). This observation, together with the finding that the mutant phenotypes are exactly the same in wild-type plants cultured in the presence of the auxin polar transport inhibitor NPA, led to the conclusion that the primary function of the pin1 gene is auxin polar transport. Using multiply marked tester strains the pin locus was mapped and found to be 3.0 +/- 0.4 map units below crabs claw which in turn is approximately 0.2 map units below Apetala on chromosome 1 of Arabidopsis thaliana (Bennett et al. (1995), The Plant J. 8, 505-520; Okada and Shimura in: Bowman J. (ed.) (1994), Arabidopsis, an Atlas of Morphology and Development, Springer Verlag Berlin, New York). However, although several attempts were made over the last years in order to isolate the pin1 gene by chromosome walking or other cloning strategies, none was successful.

Other approaches for modifying hormone controlled processes in plants have been reported. US-A-4401454, for example, discloses effects on the regulation of plant growth, including increased yields, enhanced auxin activity, inhibition of terminal growth, etc., by application of phosphonic acid derivatives. Similarly, EP-A-215445 relates to new pyrazoloisoquinoline derivatives which have auxin transport inhibiting activity and can be applied to plants as plant growth regulators to control plant height or to control abscission. WO 96/09755 describes a method for obtaining plants with delayed or inhibited fruit ripening and/or vegetable tissue senescence comprising selection and breeding of plants to produce strains with low polar auxin transport and low inhibition of cytokinin conjugation.

Also, genetic approaches for the manipulation of developmental processes in plants have been reported. WO 94/10831 discloses the transformation of plants with a DNA sequence encoding a group 3 late embryogenesis abundant (LEA) protein and the production of shorter, stronger and more branched plants. WO 92/02622 relates to plants transformed with a gene complex from melon and resulting delay in fruit ripening. EP-A-445658 describes the isolation of a IAA synthetase gene from Enterobacter cloacae which could be introduced into microorganisms or plants. WO 96/00291 discloses the expression of UDP-glucose indol-3-ylacetyl-glucosyl transferase (IAGlu Transferase) DNA in transgenic plants. By use of these non-naturally occuring nucleotide sequences, the gene product thereof being involved in the conjugation of IAA with carbohydrates, control of IAA levels and plant growth is said to become possible.

However, so far no gene or gene product directly involved in, or even essential to, polar auxin transport has been characterized. Since the pin gene product has a direct influence on, inter alia, structure, development, initiation and differentiation of the vascular system (and thereby is likely to affect sink-source relationships), plant growth, flowering, fruit ripening, abscission and cambial differention, the nucleotide sequences of the invention can be used to manipulate one or more of these features in transgenic plants.

The pin1 gene has now, however, surprisingly been successfully cloned, characterized and sequenced, as described hereinafter. Detailed descriptions of the transposon tagging strategy used, the pin1 gene and its gene product are given below. Further, the pin2 gene from A. thaliana has also been cloned using the same transposon tagging strategy. The nucleotide sequence of the pin2 gene is also given below. These nucleotide sequences now provide the possibility to manipulate several different processes of growth and development in higher plants which are controlled by auxin directly or indirectly.

Thus, one object of the invention is to provide a tool for modifying and controlling the synthesis of plant fibres. Since the pin gene product controls initiation and differentiation of the vascular system, fibre synthesis in plants is expected to be directly influenced by modifying pin gene expression. Plant fibres, being a renewable resource, are used in the production of pulp, paper, textiles and the like. Further, plant fibres are used in fibre reinforced composites, construction and building materials, (biodegradable) geotextiles, nonwovens, insulation materials and asbestos replacing materials. Fibres can be further used in mixture yarns with cotton, silk or synthetics. Cellulose from fibre crops may be successfully applied as raw material for viscose production. By manipulation of pin gene expression in plants it becomes possible to improve the control of yield and quality of plant fibres.

Another object of the invention is to improve wood quality and wood yield by regulation of wood formation. Since cambial growth is controlled by auxin through morphogenetic regulation of vascular tissues, wood production is expected to be efficiently influenced by manipulating polar auxin transport in plants. Thus, as a result of altered pin gene expression in transgenic plants, cambial division and therefore wood formation can be inhibited or enhanced, e.g. in woddy dicotyledons and gymnosperms.

Another object of the invention is to engineer novel plant architectural traits in plants. The ability to manipulate branching has numerous applications in both agriculture and horticulture. For example, reduced branching in apple trees would lead to improvements in mechanical harvesting of fruit, whereas improved root colonisation in commercial tree plantations would have benefits for both soil and tree stability. While the pattern of lateral root initiation is further central to the efficient exploitation of water and nutrient supplies, the degree of shoot branching affects important factors such as light harvesting and fecundity. The ability to control branch development and to modify branching pattern is therefore a powerful tool for plant breeders. Further, reduced branch development would lead to improvements in pulping efficiency. The regulation of plant architecture, e.g. in poplar trees, would ensure a more reproducible product for the construction industry. Producing synchronous branching, which would result in a reduction of yield loss, is also desired by plant breeders, e.g. in oilseed rape.

Other objects of the invention are based on further characteristics of plants which are influenced directly or indirectly by polar auxin transport and auxin levels. For instance, one object is to enhance parthenogenetic fruit production, e.g. in tomato and cucumber. Since parthenogenesis is stimulated by IAA and also some synthetic auxins, enhanced expression of the nucleic acid sequences of the invention, being either systemic, tissue-specific, developmental specific and/or inducible by biotic or abiotic stimuli, could satisfy this need in plant breeding.

Further, there is a need to make harvesting or picking of fruits, for example citrus and malaceous fruits, easier and more efficient. Since the force with which a plant holds on to its fruit, is also affected by auxin levels, this problem could also be solved by manipulation of polar auxin transport in plants due to accordingly tailor-made expression of the DNA sequences of the invention in transgenic plants.

The influence of IAA and auxins in general on root formation has already be mentioned. Therefore, the object of stimulating root formation, e.g. in cuttings, can also be attained by the manipulation of auxin transport and auxin levels by use of the DNA sequences of the invention.

Another object in agriculture and horticulture is to influence fruit ripening, fruit colour and/or senescence. These traits can also be enhanced, i.e. accelerated and/or increased, inhibited or delayed in transgenic plants which have altered polar auxin transport due to expression (e.g. overexpression, antisense-expression or controlled expression) of the DNA sequences of the invention.

Another object in agriculture is to control abscission and dropping of fruits. Depending on the kind of plantation and plant, dropping of fruits prior to harvest has sometimes to be prevented, e.g. in citrus fruits and malaceous fruits, whereas sometimes abscission is desired, e.g. in cotton plants ready for harvest. Shedding of leaves and fruits can also be controlled, i.e. for instance stimulated or delayed, by specific expression of the DNA sequences of the invention in transgenic plants.

Until now, plant breeders influence, stimulate or inhibit some of the above-mentioned traits by local or systemic application of auxins or polar auxin transport inhibitors to plants. However, this application is time- and energy-consuming, labour-intensive and often also cost-intensive.

It is, therefore, an object of this invention to provide a tool for the manipulation of the above mentioned traits in plants by control of polar auxin transport, in particular, to provide nucleotide sequences encoding the pin gene product and DNA molecules containing these sequences and to provide the proteins or protein fragments encoded by them. Further objects of this invention are the provision of nucleic acid molecules comprising the coding region of the pin1 gene or pin2 gene or fragments thereof, the provision of vectors and microorganisms, the uses of which open the possibility to produce new plants which have altered polar auxin transport activity and altered traits influenced by auxin transport and auxin levels, and the provision of a method for producing new plants which exhibit an altered polar auxin transport and altered auxin dependent traits in comparison to wild-type plants.

These and other objects are attained by the subject matters defined in the attached independent claims. Preferred embodiments are notable from the dependent claims and the present description.

The invention provides DNA sequences that code for proteins which are involved in polar auxin transport. Further the invention provides DNA sequences that code for proteins which are essential for polar auxin transport activity. Further the invention provides the pin gene product and predicted characteristics of this protein. As will be described below, our studies strongly support the idea that the pin protein is located to the plasma membrane and/or associated with the cytoskeleton and further is an auxin receptor protein and/or an auxin carrier protein and/or in contact with an auxin receptor protein and/or an auxin carrier protein. Without intending to be bound by any scientific theory or explanation, the invention thus also provides DNA sequences that code for proteins which control the activity of the auxin efflux carrier proteins or of other proteins involved in polar auxin transport.

In particular, the invention provides a full size cDNA clone (pcpin1/23) of the pin1 gene from Arabidopsis thaliana.

The invention further provides a full size cDNA clone (pcpin2/4) of the pin2 gene from Arabidopsis thaliana.

The cDNA clone of the pin1 gene was transformed in Escherichia coli and the E. coli strain containing the cDNA clone pcpin1/23 was deposited at the Deutschen Sammlung von Mikroorganismen (DSM), Mascheroder Weg 1B, D-38124 Braunschweig, Germany, in accordance with the requirements of the Budapest Treaty, on June 14, 1996 and received the deposit number DSM 10712.

Further, the invention provides preferably the protein encoded by the cDNA clone pcpin1/23 as well as the protein encoded by the cDNA clone pcpin2/4. These proteins are directly involved in polar auxin transport and appear to be auxin-specific receptors and/or auxin-specific efflux carrier proteins. The characteristics of the protein encodod by the pin1 gene are given below.

In the context of this invention the pin protein is referred to as the pin gene product, which is involved in polar auxin transport in plants. As mentioned above, it is known from studies on plant mutants that plants which are impaired in polar auxin transport have a "pin" shape and are often referred to as "pin"-formed mutants.

Further this invention provides DNA sequences and nucleic acid molecules that (i) hybridize to the DNA sequences of the invention or fragments thereof, (ii) are allelic derivates of the DNA sequences of the invention or fragments thereof, and/or (iii) are DNA sequences which differ from the DNA sequences of the invention or fragments thereof as a result of the degeneracy of the genetic code.

In the context of this invention "active fragment" means in general that the fragment is sufficient for influencing polar auxin transport in plants. In detail, "active fragment" refers to fragments that are capable to provide altered plant traits through overexpression, antisense-expression or controlled expression of the fragments.

The nucleic acid molecules of the invention can be any form of nucleic acid, in particular DNA or RNA molecules, e.g. cDNA, genomic DNA, mRNA, etc. The nucleic acid molecules can be naturally occuring molecules or they can be produced by chemical processes or by methods of molecular biology known to a person skilled in the art.

The invention also provides nucleic acid molecules in which the DNA sequences of the invention are combined with regulatory elements that provide their transcription and, if desired, their translation in the plant cell. Provision of the DNA sequences and nucleic acid molecules of the invention opens the possibility to manipulate plant cells, plant parts or whole plants by genetic engineering to exhibit altered polar auxin transport activity or auxin activity.

Further, the invention provides nucleic acid molecules that provide tissue-specific, development specific, inducible and/or systemic expression of the DNA sequences of the invention. It is particularly adventageous that the DNA sequences of the invention can be expressed in a controlled fashion, i.e. dependent on the trait that is influenced by polar auxin transport and which is to be enhanced or reduced, i.e. the DNA sequences of the invention can be expressed in transgenic plants in a "tailor-made" fashion. In general, polar auxin transport or auxin activity can be increased, e.g. by overexpression or regulated expression of the DNA sequences of the invention, or it can be reduced by antisense-expression or other means that reduce homologous gene expression and/or polar auxin transport activity.

The invention further provides vectors and microorganisms which contain DNA sequences and nucleic acid molecules, respectively, that code for proteins directly involved in auxin transport or auxin activity. The invention thus also provides vectors, in particular plasmids, cosmids, viruses, bacteriophages and other vectors used in molecular biology and genetic engineering, which contain the DNA sequences of the invention and which, if desired, can be used for transferring the DNA sequences of the invention to plants and plants cells. The invention also provides nucleic acid molecules and vectors, in which the DNA sequences of the invention are combined with regulatory sequences that provide transcription and translation in procaryotic and eucaryotic cells. If desired, the nucleic acid molecules and vectors may also contain additional enhancer sequences or other regulatory sequences. These regulatory sequences comprise also signal sequences, that provide the transport of the gene product to a specific cell compartment.

The invention also relates to microorganisms, such as bacteria, viruses, fungi, etc., which contain the DNA sequences of the invention.

The invention also provides new plants, which exhibit altered auxin transport and auxin activity and altered developmental and morphogenetic traits in comparison to wild-type plants. Some examples for traits which may be altered in plants through expression of the DNA sequences of the invention have been already mentioned. In particular, the invention provides plants which show altered plant architecture, e.g. reduced or enhanced branching, altered plant growth, e.g. reduced or enhanced height, altered fruit ripening, altered abscission, altered wood production, altered flowering and/or altered root formation.

The invention comprises transgenic plants, in the cells of which the DNA sequences of the invention are integrated in the plant genome. Further the invention comprises plants, in the cells of which the DNA sequences of the invention are located in a self-replicating system.

The plant that may be transformed with the DNA sequences or nucleic acid molecules of the invention can be any plant. Dependent on the trait desired to be altered, it may be a gymnosperm, dicotyledon or monocotyledon. Examples for gymnosperms are pine, spruce, fir, Douglas fir, Hemlock fir, larch, yew, cedar, juniper. Examples for monocotyledons are plants which belong to the genus of wheat (Triticum), barley (Hordeum), rye (Secale), oats (Avena), rice (Oryza), maize (Zea), millet (Sorghum), Panicum, Pennisetum, Setaria, sugar cane, bamboo. Examples for dicotyledons, and other plants, e.g. fibre plants, are cotton, leguminous plants, alfalfa, soybean, (oilseed) rape, tomato, potato, sugar beet, ornamentals, trees, e.g. poplar, sequoia (giant redwood), aspen, birch, eucalyptus, fruit plants and trees, e.g. apple, citrus, pineapple, pear, cherry, grape, banana, soft fruit plants, tea, coffee, cocoa plants, tobacco, sisal, hemp, coir, ramie, sun hamp, sansevieria, maur hemp, pita floja, flax, palms, silk cotton tree (kapok tree), coconut, jute, kenaf, miscanthus.

The invention also includes plant reproduction products of the plants of the invention, e.g. seeds, fruits, cuttings, tubers, rhizomes, and parts of the plants, as protoplasts, plant cells, calli or roots.

The invention further comprises host cells, in particular procaryotic and eucaryotic cells, which may be transformed or infected with a nucleic acid molecule or a vector of the invention and cells which are derived from those host cells and which contain the nucleic acid molecules or vectors of the invention. The host cells may be bacteria, fungi, plant cells and animal (mammalian) cells.

The invention provides methods for producing plants with altered auxin transport or auxin activity, and plant transformation methods which can be used to produce new plants with altered polar auxin transport and altered auxin transport or auxin dependent traits.

For the production of such new plants several different methods can be applied in accordance with this invention. On the one hand, plants or plant cells can be modified by common transformation methods known in genetic engineering to that extent that the new DNA seqences are integrated in the plant genome, i.e. stable transformants are created. On the other hand, the nucleic acid molecules of the invention, expression of which may result in altered auxin transport activity, can also be introduced in the plant or plant cell as self-replicating system. For instance, the nucleic acid molecules of the invention may be contained in a virus that infects the plant or plant cell.

The invention comprises a method for producing these new plants based on the DNA sequences of the invention and their transfer to plant cells and their expression in plants, which, if desired, may be controlled. By provision of the DNA sequences of the invention it is now possible to modify plant cells by genetic engineering so that they display altered auxin transport or auxin activity, in comparison to wild-type cells.

One method of the invention for the production of new plants which exhibit altered polar auxin transport or auxin activity as a result of expression of the DNA sequences of the invention, generally comprises the following steps:
a) manufacture of an expression vector, comprising (i) a promoter region that provides transcription and translation, (ii) at least one DNA sequence that codes for the pin protein or an active fragment thereof, wherein this DNA sequence is combined in sense orientation to the 3' end of the promotor, and if desired or necessary (iii) a termination signal for transcription termination and addition of a poly-A-tail to the respective transcript, wherein the termination signal is combined to the 3' end of the coding region;
b) transformation of plant cells with the expression vector produced in step a);
c) regeneration of transgenic plants and, if desired, breeding of the plants.

In an alternative method according to this invention, the coding region of the pin gene or fragments thereof can be combined with an promoter in such a way that they are oriented in the antisense orientation.

In another alternative inventive method, the DNA sequences of the invention can be introduced in plant cells or plants as self-replicating system.

The invention comprises uses of the genetic and protein materials, especially including the use of the new DNA sequences and molecules for the production of plants which have an altered polar auxin transport or auxin activity. Further, the invention includes the use of the new DNA sequences and molecules for the production of plants in which plant growth, architecture, branching, wood production, morphogenesis, differentiation of the vascular tissues, abscission, flowering, fruit ripening, root formation and/or other auxin controlled developmental processes are altered.

The invention includes the use of the DNA sequences of the invention or the fragments thereof and the use of the proteins of the invention or the fragments thereof for the isolation of pin genes, fragments thereof or DNA sequences which are homologous to the DNA sequences of the invention and the isolation of pin proteins, fragments thereof or proteins which are homologous to the proteins of the invention. The invention also includes the use of the DNA sequences of the invention or fragments thereof and the use of the gene product or fragments thereof for the isolation of DNA sequences that code for proteins involved in polar auxin transport.

Thus the present invention incorporates any possible use of the DNA sequences of the invention, expression of which results in altered auxin transport or auxin activity, as well as the use of the proteins of the invention or fragments thereof, which have polar auxin transport activity.

In general, any regulatory sequences which fulfils the prerequisite of providing the desired gene expression pattern in the plant of choice can be used in order to express the DNA sequences of the invention in plants and to manipulate auxin transport dependent plant traits in a tailor-made fashion. These can be constitutive promoters, tissue-specific promoters, e.g. such which provide stem-specific expression, developmental specific promoters or promoters which are induced by abiotic or biotic stimuli. One example for a constitutive promoter that can be used in the scope of the invention is the 35S RNA promoter from Cauliflower Mosaic Virus (described e.g. in Rogers et al. (1987) Meth. Enzymol. 153, 253-277). Other constitutive promoters that are active in plants are known to the person skilled in the art. Examples for tissue-specific promoters are, for instance, seed specific promoters (e.g. in Plant et al. (1994) Plant Mol. Biol. 25, 1193-1205), vascular tissue specific promoters (e.g. in Guevera-Garcia et al. (1993) The Plant J. 4, 495-505; Brears et al. (1991) The Plant J. 1, 235-244), and stem specific promoters. Examples for developmental specific promoters or hormone induced promoters are e.g. given in Vögeli-Lange et al. (1994), Plant Mol. Biol. 25, 299-311; Langridge et al. (1989) Proc. Natl. Acad. Sci. USA 86, 3219-3223. Promoters which are induced by abiotic stimuli comprise those which are stimulated by chemicals, ozone, UV light, extreme temperatures, dryness, salt, etc., and which are described e.g. in Williams et al. (1992), Biotechnology 10, 540; Schulze-Lefert et al. (1989) EMBO J. 8, 651-656; Schulze-Lefert et al. (1989) Plant Cell 1, 707-714. Promoters which are induced by biotic stimuli comprise promoters which are induced by pathogens, e.g. fungi, bacteria, viruses, insects and nematodes. Such promoters are known to the person skilled in the art and given in the literature, e.g. Martini et al. (1993) Molecular and General Genetics 236, 179-186, or WO 94/17194.

In case useful promoters are not already known, the strategy and methods for the isolation of such promoters are known to the person skilled in molecular biology. In general, in a first step poly(A)⁺ is isolated from a certain tissue, a plant or tissue of a certain developmental stage, or a plant or tissue treated with a certain stimuli, and a cDNA library is established using common means and methods of molecular biology. In parallel a second cDNA library is established from other tissues, plants or tissues of another developmental stage, or non-treated plants or tissues.

Then, in a second step, those clones are identified by differential hybridization, the corresponding poly(A)⁺ of which are only expressed in the respective tissue, during the respective developmental stage or after treatment with an biotic or abiotic stimulus. Subsequently, the cDNA clones isolated by following the described strategy are used in order to isolate the corresponding promoters, which can then be used in order to express the DNA sequences of the invention in plants. By this way, the DNA sequences of the invention can be expressed in a controlled fashion in any tissue or developmental stage, or their expression can be induced by a certain treatment, whenever expression is desired in the whole plant or in certain plant parts.

When the DNA sequences of the invention are used in order to inhibit or reduce gene expression of homologous genes in transgenic plants, the DNA sequences provided by the invention can also be expressed in the antisense orientation. Antisense expression can also be controlled by constitutive, tissue-specific, developmental specific or otherwise inducible promoter sequences.

The invention also comprises nucleic acid moleluces that code for proteins which have auxin transport activity or fragments thereof which hybridize to the DNA sequences and nucleic acid molecules of the invention. In the context of the invention, the term "hybridization" means a hybridization under convential conditions, preferably under stringent conditions, as e.g. described in Sambrook et al. (1989), Molecular Cloning: A Laboratory Manual, 2nd edition, Cold Spring Harbor Laboratory Press, Cold Spring Harbor, New York. Nucleic acid molecules that hybridize with the molecules of the invention may isolated e.g. from genomic or cDNA libraries. Identification and isolation of such nucleic molecules can be carried out using the nucleic acid molecules of the invention or fragments or reverse complements thereof, e.g. by hybridization under standard conditions (Sambrook et al., supra).

Thus the invention also comprises the use of the DNA sequences of the invention or fragments thereof for the identification and isolation of homologous sequences from plants or other organisms.

For instance, nucleic acid molecules which display exactly or essentially the nucleic acid sequences of the invention or portions thereof can be used as hybridization probes. Also synthetic fragments, which are synthesized using common synthesis processes and which correspond basically to one of the DNA sequences or nucleic acid molecules of the invention can be used as hybridization probes. When genes are identified and isolated, which hybridize to the DNA sequences of the invention, it is necessary to determine their sequence and the sequence and characteristics of the proteins encoded by them. The man skilled in the art has a variety of biochemical, biotechnological and genetic engineering methods for the characterization of the nucleic acid molecules and the proteins at his disposal.

The molecules that hybridize to the nucleic acid molecules of the invention comprise also fragments and (degenerated or allelic) derivatives of the nucleic acid molecules described herein. The terms "derivatives" means in this context that the sequences of such molecules differ from the molecules of the invention (as herein described) in one or more positions and show a high degree of homology to the sequences provides by the invention. Homology means an identity in sequence of at least 40 %, in particular of at least 60 %, preferably of more than 80 % and more preferably of more than 90%.

The deviations from the sequences of the invention may be generated by deletion, addition, substitution, insertion or recombination.

Homology further means that there is a functional and/or structural equivalence between the respective nucleic acid molecules and the proteins encoded by them. Usually the nucleic acid molecules which are homologous to the molecules of the invention and which are derivatives of these molecules, are variations or modification which have the same biological function. These variations may be naturally occuring variations, e.g. related sequences from other organisms, or they can be mutations, which may be either spontaneous or artificially introduced (e.g. by targeted mutagenesis). Further, the variants can be synthetically produced sequences. Also the allelic variants may be naturally occuring or synthetically produced variants, or variants generated by recombinant DNA techniques.

Usually the proteins encoded by the different variants and derivatives of the nucleic acid molecules of the invention have common characteristics. Such characteristics are e.g. the molecular weight, enzyme activity, efflux activity, binding capacity, immunological reactivity, conformation, etc. Further common characteristics may be physical properties, e.g. migration pattern in gel electrophoresis, chromatographic characteristics, sedimentation coefficient, solubility, spectroscopic properties, stability, pH optimum, temperature optimum, etc..

Polar auxin transport activity can be measured according to Hertel et al. (1983) Planta 157, 193-201; Parker and Briggs (1990) Plant Physiol. 94, 1763-1769; Okada et al., supra, Bennett et al., supra; general information is also given in Lomax et al. (1995) in: Davies (ed.), Plant Hormones, Kluwer Dordrecht. Thus, expression of the pin gene and activity of the pin protein can be easily measured in wild-type, transgenic and mutant plants.

For the preparation of foreign genes for their introduction into higher plants, a variety of cloning vectors are available, e.g. such vectors which have an origin of replication active in E. coli and a marker gene for the selection of transformed bacteria cells. Examples for such vectors are pBR322, several pUC-vectors, M13mp-vectors, pACYC184, etc. The desired nucleic acid sequence can be incorporated in a suitable restriction site of the vector and the obtained plasmid is used for the transformation of E. coli cells.

Transformed E. coli cells are grown in a suitable medium, subsequently harvested and lysed. The plasmid is recovered and the plasmid DNA can now be analyzed and characterized by restriction mapping, gel electrophoresis, sequencing and other biochemical and genetic engineering methods known to the person skilled in the art. After each manipulation the plasmid DNA can be digested and the obtained linearized vectors or fragments can be combined with other DNA sequences. Each plasmid DNA sequence can be cloned in the same or other plasmids.

For introducing DNA into a plant host cell several techniques are available and the person skilled in the art can easily choose a suitable transformation procedure. These techniques comprise the transformation of plant cells with T-DNA using Agrobacterium tumefaciens or Agrobacterium rhizogenes as transformation means, fusion of protoplasts, direct gene transfer of isolated DNA into protoplasts, microinjection or electroporation of DNA, introducing DNA via biolistic methods and other procedures. In an alternative embodiment of the invention the nucleic acid molecules of the invention can be introduced into plant cells via viral infection.

When the DNA is transferred by microinjection or electroporation any form of DNA may be used. The same applies for direct gene transfer. Here, simple plasmids, as for example pUC derivatives can be used. However, if whole plants are to be regenerated from transformed plant cells a marker gene which allows selection of transformed plants is necessary. Suitable selection markers are known to the man skilled in the art and he can easily choose a suitable marker.

Depending on the transformation method used further DNA sequences may be required. For instance, if the Ti- or Ri-plasmid is used, the gene to be transferred has to be combined with at least the right border of the T-DNA contained in the Ti- and Ri-plasmid. Often, the gene of interest has to be combined with both the right and the left border of the T-DNA as flanking areas.

If agrobacteria are used for the transformation, the DNA to be transferred has to be cloned into specific plasmids, i.e. into an intermediary or a binary vector. Intermediary vectors comprise sequences homologous to sequences of the T-DNA which allow them to be integrated in the Ti- or Ri-plasmid of agrobacteria by homologous recombination. Further, this plasmid carries the vir-region which is required for the T-DNA transfer. Intermediary vectors are not capable to replicate in agrobacteria. The intermediary vector can be transferred to agrobacteria via a helper plasmid (conjugation). Binary vectors are able to replicate in both E. coli and agrobacteria. They contain a selection marker gene and a linker or polylinker framed by the left and right border regions of the T-DNA. These vectors can be directly transformed into agrobacteria (Holsters et al. (1978), Molecular and General Genetics 163, 181-187). The agrobacterial host cell should contain a plasmid carrying the vir-region which is necessary for the transfer of the T-DNA into the plant cell. Addition T-DNA may be present.

The transformed agrobacterium is used for the transformation of plant cells. The use of T-DNA for the transformation of plant cells is well-studied and suffiently described in EP 120 515; Hoekema in: The Binary Plant Vector System, Offsetdrokkerij Kanters B.V., Alblasserdam (1985) Chapter V; Fraley et al. (1993), Crit. Rev. Plant Sci. 4, 1-46 and An et al. (1985), EMBO J. 4, 277-287.

For the transfer of DNA into plant cells plant explants can be effectively co-cultivated with Agrobacterium tumefaciens or Agrobacterium tumefaciens. Subsequently, whole plants can be regenerated from the infected plant material (e.g. leaves or leaf pieces, stem segments, roots, protoplasts or plant cells cultivated in suspension cultures) in a suitable medium containing antibiotica or biocides as selection agents. The regeneration of the plants is accomplished by common regeneration methods using known nutrient media. The obtained plants can then be analyzed for the DNA that was to be transferred using standard biochemical and genetic engineering methods. Biolistic transformation methods can also be applied, as described e.g. in Willmitzer (1993) Transgenic Plants, in: Biotechnology, A Multi-Volume Comprehensive Treatise (H.J. Rehm, G. Reed, A. Pühler, P. Stadler, eds) Vol. 2, 627-659, V.C.H. Weinheim - New York - Basel - Cambridge).

While the transformation of dicotyl plants via A. tumefaciens using Ti-plasmid-vector systems is well established, recent studies seem to indicate that also monocotyl plants can be transformed via Agrobacterium-derived vectors (Chan et al. (1993), Plant Mol. Biol. 22, 491-506; Hiei et al. (1994), Plant J. 6, 271-282; Deng et al. (1990), Science in China 33, 28-34; Wilmink et al. (1992), Plant Cell Reports 11, 76-80; May et al. (1995) Bio/Technology 13, 486-492; Conner and Domiss (1992) Int. J. Plant Sci. 153, 550-555; Ritchie et al. (1993) Transgenic Res. 2, 252-265).

Alternative systems for the transformation of monocotyledons are procedures using the biolistic approach (Wan and Lemaux (1994), Plant Physiol. 104, 37-48; Vasil et al. (1993), Bio/Technology 11, 1553-1558; Ritala et al. (1994), Plant Mol. Biol. 24, 317-325; Spencer et al. (1990), Theor. Appl. Genet. 79, 625-631), transformation of protoplasts, electroporation of partially permeabilized cells, the introduction of DNA via glass fibres.

Transformation methods that can be used for the transformation of gymnosperms are described e.g. in Liu et al. (1993) Plant Mol. Biol. 23, 297-308; Primich and Minocha (1991) Plant Cell Reports 10, 545-549; Morris et al. (1989) Phys. Mol. Plant Pathol. 34, 451-462.

Once the introduced DNA is integrated into the plant genome, it remains stable and is also stably inherited to the progeny of the originally transformed plant cell. Usually, it contains a selection marker conferring resistance against a biocide or antibioticum as kanamycin, G418, bleomycin, hygromycin, methotrexate, glyphosate, streptomycin, sulphonyl urea, gentamycin, phosphinotricin, etc. The selection marker which can be chosen individually should therefore allow selection of transformed cells over cells which are devoid of the transferred DNA.

The transformed cells grow within the plant in the normal way (e.g. McCormick et al. (1986) Plant Cell Reports 5, 81-84). The resulting plants can be cultivated in the usual fashion and may be propagated by self-fertilization or be crossed with plants having the same transformed or other hereditary factors. The hybrids obtained by crossings have the respective phenotypic characteristics. Usually, seeds can be obtained from the plants.

Two or more generations should be grown in order to ensure that the phenotypic trait is stably maintained and inherited. Also transgenic lines can be selected by usual methods which are homozygous for the new nucleic acid molecules. Heterozygous and homozygous plants can then be analyzed for their auxin transport and auxin activity and their phenotypic behaviour in respect to vascular tissue development, wood production and structure, juveniliy, branching, and other traits which are influenced directly by polar auxin transport and which can be manipulated within the scope of this invention.

The isolation and charcterisation of the pin genes using a transposon tagging strategy is described in the following examples which are meant to be illustrative but not limiting.

### Description of the Figures

Figure 1 shows the pKEn2 construct what was transformed into the Arabidopsis plants. BL: left border of the T-DNA; BR: right border of the T-DNA; HPT: hygromycine phosphotransferase gene; P35S: Cauliflower Mosaic Virus 35S promoter; P1':TR-DNA 1' promoter; P: En promoter; NPTII: neomycine phosphotransferase II gene (kanamycine resistance gene).

Figure 2 depicts the En insertion site in the pin1 cDNA.

Figure 3 shows the open reading frame (ORF) of the pin1 cDNA clone pcpin1/23 (SEQ:NO 11).

Figure 4 depicts the predicted characteristics of the pin1 protein with respect to the hydrophilic and hydrophobic characteristics and the surface probability. The plots are made by DNAstar, a computer program DNA analysing program using the Kyte-Doolittle and the Emini algorythms.

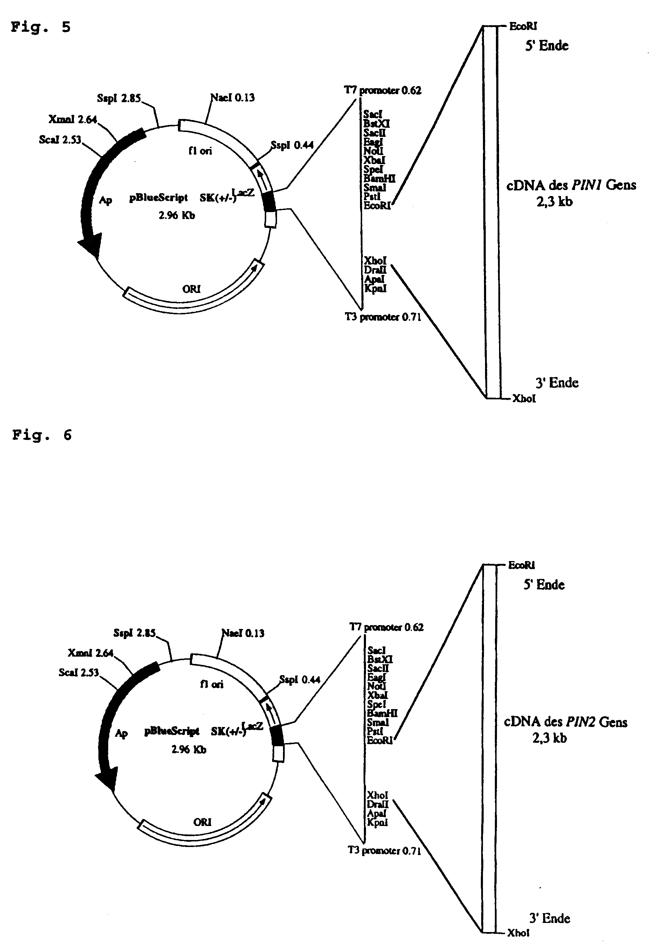

Figure 5 shows the plasmid map of pcpin1/23. The cDNA of the pin1 gene, contained on a 2.3 kb EcoRI/XhoI fragment was inserted between the EcoRI and XhoI restriction sites of the pBlueScript SK(+/-) vector.

Figure 6 shows the plasmid map of pcpin 2/4. The cDNA of the pin2 gene, contained on a 2.3 kb EcoRI/XhoI fragment was inserted between the EcoRI and XhoI restriction sites of the pBlueScript SK(+/-) vector.

Sequence list

SEQ:NO 1        En 6988F

5'-GCA GAT GTA TGG AGG TGC CGG AAC-3'

SEQ:NO 2        En 7635R

5'-CGA GTG TAG TAC GAT CAG GCG GAG-3'

SEQ:NO 3        En 578R

5'-AGG TTG GGC AGA AAC ATT TTG CTT-3'

SEQ:NO 4        APL16

5'-phosphorylated-TA TGG GAT CAC ATT AA-3'-amino

SEQ:NO 5        LR 32

5'-ACT CGA TTC TCA ACC CGA AAG TAT AGA TCC CA-3'

SEQ:NO 6        En 205R

(biotin-)5'-AGA AGC ACG ACG GCT GTA GAA TAG GA-3'

SEQ:NO 7        LR 26

5'-ACT CGA TTC TCA ACC CGA AAG TAT AG-3'

SEQ:NO 8        En 91R

5'-TGC AGC AAA ACC CAC ACT TTT ACT TC-3'

SEQ:NO 9        En 48R

5'-TTT ACT TCG ATT AAG AGT GTC GGC C-3'

SEQ:NO 10       En 1R

5'-ACT CCT TTG ACG TTT TCT TGT AGT G-3'

SEQ:NO 11       cDNA sequence of the pin1 gene (pcpin1/23)

1   GTCTCGAGTT TTTTTTTCTT TTTTCCCTCT CACCACTTCT CTCTCAAACT
51  AAAGACAAAA GCTCTTCTCT CTTCCCTCTC TCTTCTCCGG CGAACAAAAA
101 GATGATTACG GCGGCGGACT TCTACCACGT TATGACGGCT ATGGTTCCGT
151 TATACGTAGC TATGATCCTC GCTTACGGCT CTGTCAAATG GTGGAAAATC
201 TTCACACCAG ACCAATGCTC CGGCATAAAC CGTTTCGTCG CTCTCTTCGC
251 CGTTCCTCTC CTCTCTTTCC ACTTCATCGC CGCTAACAAC CCTTACGCCA
301 TGAACCTCCG TTTCCTCGCC GCAGATTCTC TCCAGAAAGT CATTGTCCTC
351 TCTCTCCTCT TCCTCTGGTG CAAACTCAGC CGCAACGGTT CTTTAGATTG
401 GACCATAACT CTCTTCTCTC TCTCGACACT CCCCAACACT CTAGTCATGG
451 GGATACCTCT TCTCAAAGGC ATGTATGGTA ATTTCTCCGG CGACCTCATG
501 GTTCAAATCG TTGTTCTTCA GTGTATCATT TGGTACATAC TCATGCTCTT
551 TCTCTTTGAG TACCGTGGAG CTAAGCTTTT GATCTCCGAG CAGTTTCCAG
601 ACACAGCAGG ATCTATTGTT TCGATTCATG TTGATTCCGA CATTATGTCT
651 TTAGATGGAA GACAACCTTT GGAAACTGAA GCTGAGATTA AAGAAGATGG
701 GAAGCTTCAT GTTACTGTTC GTCGTTCTAA TGCTTCAAGG TCTGATATTT
751 ACTCGAGAAG GTCTCAAGGC TTATCTGCGA CACCTAGACC TTCGAATCTA
801 ACCAACGCTG AGATATATTC GCTTCAGAGT TCAAGAAACC CAACGCCACG
851 TGGCTCTAGT TTTAATCATA CTGATTTTTA CTCGATGATG GCTTCTGGTG
901 GTGGTCGGAA CTCTAACTTT GGTCCTGGAG AAGCTGTGTT TGGTTCTAAA

951 GGTCCTACTC CGAGACCTTC CAACTACGAA GAAGACGGTG GTCCTGCTAA
1001 ACCGACGGCT GCTGGAACTG CTGCTGGAGC TGGGAGGTTT CATTATCAAT
1051 CTGGAGGAAG TGGTGGCGGT GGAGGAGCGC ATTATCCGGC GCCGAACCCA
1101 GGGATGTTTT CGCCCAACAC TGGCGGTGGT GGAGGCACGG CGGCGAAAGG
1151 AAACGCTCCG GTGGTTGGTG GGAAAAGACA AGACGGAAAC GGAAGAGATC
1201 TTCACATGTT TGTGTGGAGC TCAAGTGCTT CGCCGGTCTC AGATGTGTTC
1251 GGCGGTGGAG GAGGAAACCA CCACGCCGAT TACTCCACCG CTACGAACGA
1301 TCATCAAAAG GACGTTAAGA TCTCTGTACC TCAGGGGAAT AGTAACGACA
1351 ACCAGTACGT GGAGAGGGAA GAGTTTAGTT TCGGTAACAA AGACGATGAT
1401 AGCAAAGTAT TGGCAACGGA CGGTGGGAAC AACATAAGCA ACAAAACGAC
1451 GCAGGCTAAG GTGATGCCAC CAACAAGTGT GATGACAAGA CTCATTCTCA
1501 TTATGGTTTG GAGGAAACTT ATTCGTAATC CCAACTCTTA CTCCAGTTTA
1551 TTCGGCATCA CCTGGTCCCT CATTTCCTTC AAGTGGAACA TTGAAATGCC
1601 AGCTCTTATA GCAAAGTCTA TCTCCATACT CTCAGATGCA GGTCTAGGCA
1651 TGGCTATGTT CAGTCTTGGG TTGTTCATGG CGTTAAACCC AAGAATAATA
1701 GCTTGTGGAA ACAGAAGAGC AGCTTTTGCG GCGGCTATGA GATTTGTCGT
1751 TGGACCTGCC GTCATGCTCG TTGCTTCTTA TGCCGTTGGC CTCCGTGGCG
1801 TCCTCCTCCA TGTTGCCATT ATCCAGGCAG CTTTGCCGCA AGGAATAGTA
1851 CCGTTTGTGT TTGCCAAAGA GTATAATGTG CATCCTGACA TTCTTAGCAC
1901 TGCGGTGATA TTTGGGATGT TGATCGCGTT GCCCATAACT CTTCTCTACT
1951 ACATTCTCTT GGGTCTATGA AGAGATATTA CCAAAACACA GGGACTTTGT
2001 TTTATTCTTT TGTGGGATGA TGAATTGTGA AAAGAACAAT GCCCTTTTTG
2051 TTGAAAACCC ACAAATTAAA TCAGAAGCAG CTTTAGAGAA TCTTTGAGGA
2101 TAATTGAAGC TCTTGAAGAA GAGAAGAAGA AGGAGACTTA AGTAGGAGCT
2151 CAGCAAGTTT TACCTTTTTC TTAATTTTAA TGAACATTCG TGTTTCCTCT
2201 TTTGGTAGGT TTTAGGAATT TGTAAAAGCT TTGGCTACTT TTAGTGAATT
2251 AAAAACGTTA AGGAAAATAT CAAAAAAAAA AAAAAAAAAA AA

SEQ:NO 12 cDNA sequence of the pin2 gene (pcpin2/4)

1 GGCACGAGCC GGTTCGTTGC GGTTTTCGCG GTTCCTCTTC TCTCTTTCCA
51 TTTCATCTCC TCCAATGATC CTTATGCAAT GAATTACCAC TTCCTCGCTG
101 CTGATTCTCT TCAGAAAGTC GTTATCCTCG CCGCACTCTT TCTTTGGCAG
151 GCGTTTAGCC GCAGAGGAAG CCTAGAATGG ATGATAACGC TCTTTTCACT
201 ATCAACACTG CCTAACACGT TGGTAATGGG AATCCCATTG CTTAGGGCGA

251 TGTACGGAGA CTTCTCCGGT AACCTAATGG TGCAGATCGT GGTGCTTCAG
301 AGCATCATAT GGTATACATT AATGCTCTTC TTGTTTGAGT TCCGTGGGGC
351 TAAGCTTCTC ATCTCCGAGC AGTTCCCGGA GACGGCTGGT TCAATTACTT
401 CCTTCAGAGT TGACTCTGAT GTTATCTCTC TTAATGGCCG TGAACCCCTC
451 CAGACCGATG CGGAGATAGG AGACGACGGA AAGCTACACG TGGTGGTTCG
501 AAGATCAAGT GCCGCCTCAT CAATGATCTC TTCATTCAAC AAATCTCACG
551 GCGGAGGACT TAACTCCTCC ATGATAACGC CGCGAGCTTC AAATCTCACC
601 GGCGTAGAGA TTTACTCCGT TCAATCGTCA CGAGAGCCGA CGCCGAGAGC
651 TTCTAGCTTT AATCAGACAA ATTTCTACGC AATGTTTAAC GCAAGCAAAG
701 CTCCAAGCCC TCGTCACGGT TACACTAATA GCTACGGCGG CGCTGGAGCT
751 GGTCCAGGTG GAGATGTTTA CTCACTTCAG TCTTCTAAAG GCGTGACGCC
801 GAGAACGTCA AATTTTGATG AGGAAGTTAT GAAGACGGCG AAGAAAGCAG
851 GAAGAGGAGG CAGAAGTATG AGTGGGGAAT TATACAACAA TAATAGTGTT
901 CCGTCGTACC CACCGCCGAA CCCAATGTTC ACGGGGTCAA CGAGTGGAGC
951 AAGTGGAGTC AAGAAAAAGG AAAGTGGTGG CGGAGGAAGC GGTGGCGGAG
1001 TAGGAGTAGG AGGACAAAAC AAGGAGATGA ACATGTTCGT GTGGAGTTCG
1051 AGTGCTTCTC CGGTGTCGGA AGCCAACGCG AAGAATGCTA TGACCAGAGG
1101 TTCTTCCACC GATGTATCCA CCGACCCTAA AGTTTCTATT CCTCCTCACG
1151 ACAACCTCGC TACTAAAGCG ATGCAGAATC TGATAGAGAA CATGTCACCG
1201 GGAAGAAAAG GGCATGTGGA AATGGACCAA GACGGTAATA ACGGGGGAAA
1251 GTCACCTTAC ATGGGCAAAA AAGGTAGCGA CGTGGAAGAC GGCGGTCCCG
1301 GTCCTAGGAA ACAGCAGATG CCGCCGGCGA GTGTGATGAC GAGACTAATT
1351 CTGATAATGG TTTGGAGAAA ACTCATTCGA AACCCTAACA CTTACTCTAG
1401 TCTCTTTGGC CTTGCTTGGT CCCTTGTCTC TATCAAGTGG AATATAAAGA
1451 TGCCAACGAT AATGAGTGGA CCGATTTCGA TATTATCTGA TGCTGGTCTT
1501 GGAATGGCTA TGTTTAGTCT TGGTCTATTT ATGGCATTGC AACCAAAGAT
1551 TATTGCGTGC GGAAAATCAG TAGCAGGGTT TGCGATGGCC GTAAGGTTCT
1601 TGACTGGACC AGCCGTGATC GCAGCCACCT CAATAGCAAT TGGTATTCGA
1651 GGTGATCTCC TCCATATCGC CATCGTTCAG GCTGCTCTTC CTCAAGGAAT
1701 CGTTCCTTTT GTTTTCGCCA AAGAATATAA CGTCCATCCT GATATTCTCA
1751 GCACTGCGGT TATATTCGGA ATGCTGGTTG CTTTGCCTGT AACAGTACTC
1801 TACTACGTTC TTTTGGGGCT TTAAGTTATT ATCAAAACGT ATTTGCAAAT
1851 AAAAGGCGAT ACGACCCAAA GGTGATTTTT TTTCAAACGA AAAAGAATAA
1901 TTACAAGAAC GAAAAAAGAC TAATTCCAGG TCAGAGGCTT AGGTGTATGG
1951 GACCATGCAA TGTCGCATTA ATTAAATTAT AGCATATGAT AGTCGAAAAT
2001 TTAGATAACT TTGTATAATT AATTATATGC ACATGCATGT ACGTGACTTT

2051 GTAGTTTTTG TTACATTTAT TAAATTTTTG GGATGTGCAA GTACAATTAT
2101 TACTAAAAAA AAAAAAA

### EXAMPLE I

### Generation of En transposon mutagenised Arabidopsis thaliana population

### 1. Vector for introduction of the heterologous transposon En into Arabidipsis thaliana

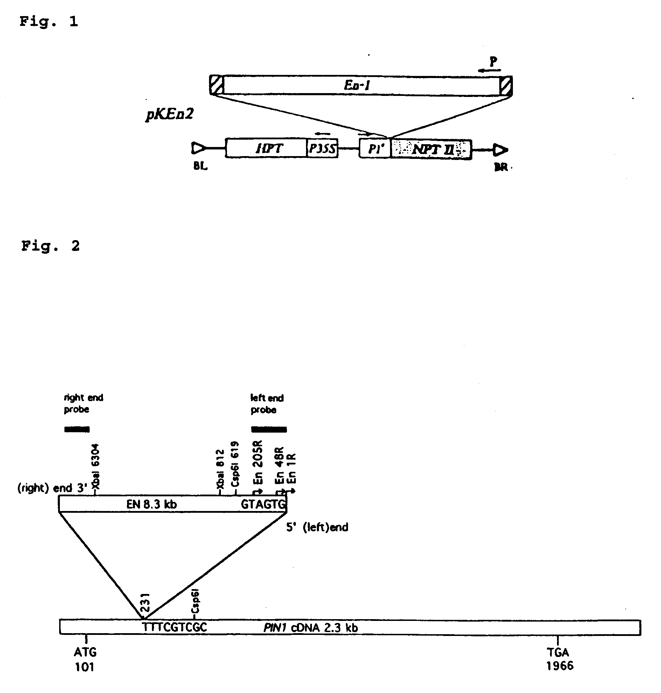

The heterologous En transposon (described in Pereira et al. (1986), EMBO J. 5, 835-841) was integrated into the genome of Arabidopsis thaliana using the agrobacterial plant transformation system. pKEn2 (Fig. 1) was the binary transformation vector that carried the En transposable element on the T-DNA. Detailed information about the plasmid pKEn2 it given in Frey et al. (1990), EMBO J. 9, 4037-4044 and Cardon et al. (1993), Plant J. 3, 773-784).

### 2. Plant material and transformation

Roots of A. thaliana (L.) Heynh ecotype Columbia (Columbia "0") were transformed with pKEn2 by Agrobacterium tumefaciens using the protocol described by Valvekens et al. (1988), Proc. Natl. Acad. Sci. USA 85, 5536-5540. Root explants were obtained from plants growing axenically on solid or liquid GM medium (Valvekens et al. supra). For culture in liquid medium, 2-week-old seedlings were transferred to 250 ml Erlenmayer flasks containing 75 ml GM medium and incubated on a rotatory shaker at 100 r.p.m. for 3 weeks. Growth of A. tumefaciens after co-cultivation was inhibited with 400 mg/l claforan (available from Hoechst, Frankfurt, Germany). This amount was reduced stepwise with every passage to fresh medium (minus 100 mg/l every 2 weeks) until a final concentration of 100 mg/l was reached. Transformed cells, as well as the progeny of transformed plants, were selected with 50 mg/l kanamycin monosulphate (available from Sigma, München, Germany), 15 mg/l hygromycin B (available from Duchefa, Haarlem, Netherlands) or 50 µg/l methotrexate (available from Sigma, München, Germany). The number of transformed loci was estimated by the segregation ratio of the marker gene linked to the T-DNA and later confirmed by Southern analysis (Sambrook et al., supra).

### 3. Origin of the En pin1 mutant plant material

- T0:: - A. thaliana ecotype Columbia "0". Root material of this plant was transformed with A. tumefaciens as described above. The binary transformation vector carrying the En transposon on its T-DNA was pKEn2 (see Fig. 1).
- T1:: - 24-2A, single copy primary transformant, hygromycin (hyg) resistant
- T2:: - self of 24-2A
- 26 plants homozygous for hyg resistance (NPTII::En/NPTII::En),
- 20 plants hemizygous for hyg resistance (NPTII::En/wt) (wt = wild-type)
- about 7.5 % were germinal revertants carrying a transposon En or not
- T3:: - selfs (about 3900 plants) of the 46 T2 plants
- selection on 300 to 400 mg/l kanamycin (km) and screening for germinal revertants (350 revertants)
- genotypes NPTII::En/NPTII, NPTII/NPTII or NPTII/wt
- germinal excision of En in Arabidopsis it about 7.5 %
- T4:: - selfs of 350 revertants of T3 (100 to 200 seeds per revertant)
- screen for insertion mutants, transposed and newly inserted Ens (Southern blot analysis)
- cross to wild-type Arabidopsis using pollen from the revertant plants 293, 325, 328, 330 to outcross the T-DNA, aim: outcross of T-DNA, the transposon remaining active in the plant
- T5:: - F1 generation of the cross: wt x 292 / 325 / 328 / 330,
- PCR screen for plants carrying a transposed En and no T-DNA, but the plants had all the NPTII gene (tEn/wt, NPTII/wt),
- another selfing
- T6/S0:: - selfs of T5 plants = F2 generation of the cross: wt. x germinal revertants
- PCR screen for plants carrying a transposed En and no T-DNA
- positive plants: G11 (line 325)
   G24, G37, G38 (line 328)
   G48, G54 (line 330)
   G69 (line 293)
- genotype: tEn/wt or tEn/tEn, no NPTII gene
- S1:: - 100 selfs of G11, G24, G37, G48, and G69 (100 seeds of each plant sawn, in total 500 S1 plants
- S2:: - single seed descendents of the 500 selfed plants of S1
- S3:: - single seed descendents of the 500 selfed plants of S2
- S4:: - single seed descendents of the 500 selfed plants of S3
- S5:: - single seed descendents of the 500 selfed plants of S4
- S6:: - single seed descendents of the 500 selfed plants of S5
- 150 plants analysed on Southern blot
- S7:: - 14 seeds (selfs) of 70 G24 S6 single seed descendents sawn
- in one out of the 70 (14 seed) pools, the one of which the parent plant is 5673-2, the En pin1 mutant is detected
- > 5673-2 (S6) is hemizygous for the pin1 mutation

### EXAMPLE II

### Screening and identification of a pin1 mutant in the En mutagenised population

### 1. Detection and identification of the pin1 mutant: characteristics

The pin1 mutant was detected by its obvious, morphological phenotype, that has already described in literature (Okada et al., supra). The detected En transposon pin1 mutant exhibited a striking similarity to the pin-formed 1-1 mutant, derived from the Enkheim ecotype and to the pin-formed 1-2 mutant, isolated from ethyl methanesulfonate (EMS) mutagenised seeds of the Landsberg ecotype (Okada et al., supra). As these mutants the En transposon pin1 mutant showed structural and morphological abnormalities in the inflorescence axes, flowers, leaves and cotelydons. The most obvious phenotype is the pin-shaped inflorescence with no flowers or floral buds at all or few deformed and sterile flowers, or just some pistil like tissue on top of it. When flowers or flower like organs are formed, the petals are abnormally shaped, no stamens with pollen are formed and the pistil is usually formed with abundant papillae tissue, but fertilisation can not take place. Some of the leaves of that pin1 mutant become much wider and attain a quite deformed and distorted shape. In some cases the major vein is branched at the base of the leaf. Some transposon pin1 mutants have three cotyledons whereas in other cases the cotelydons are fused or deformed.

### 2. Mendelian Segregation of the pin1 phenotype

126 siblings (S7 of 5673-2) of the identified En transposon pin1 mutant were sawn out in the greenhouse and the following segregation pattern was observed:
95 wild type : 31 pin1 phenotype (3,06 : 1,0)
This ratio approximates quite reasonably the Mendelian segregation ratio of 3:1 for the inheritance of a recesstve genetic trait in a selfing experiment. The fact that the pin1 mutation is recessive has already been reported in the literature.

### 3. Southern blot analysis of the pin1 segregating population using En specific probes

Genomic DNA of 37 plants of the pin1 phenotype segregating population (12 pin1 phenotpyes and 25 wild-type phenotypes) was applied to Southern hybridization experiments using a right end and a left end radioactively labelled probe from the En transposon base pair sequence. The En element contains two XbaI restriction sites, one around 800 bp from the 5' end (left end) and the other about 2,000 bp from the 3' end (right end). The genomic DNA was therefore restricted with XbaI and En elements integrated into the plant genomes could be detected by 2 specific probes:
- En right end probe: En DNA amplified by PCR with primers:
   En 6988F (SEQ NO:1) and En 7635R (SEQ NO:2).
- En left end probe: En DNE amplified by PCR with primers:
   En 578R (SEQ NO:3) and universal primer (available from Pharmacia, Freiburg, Germany).

- Results:: - integration of several (2-7) transposons into the Arabidopsis genome
- among the 25 wild-type phenotypes 15 plants are heterozygous for the En mutated pin1 allel
- correlation between the pin1 phenotype and 1 En band (pin1 typic) indicating that the mutant is transposon tagged

### EXAMPLE III

### Isolation of the En flanking DNA by the transposon insertion display (TID) procedure

### 1. Digestion and purification of pin1 mutant genomic DNA

To isolate the flanking DNA of the pin1 typic En element 1 µg of genomic DNA of Enpin1 mutant plants was digested with the restriction enzmye Csp6I (available from New England Biolabs, Schwalbach, Germany) in parallel with wild-type plants as controls. The genomic plant DNA flanking the left end (5' end) of the pin1 typic En transposon was isolated via a linker mediated PCR procedure. The Csp6I restriction digest produced a DNA fragment with a convenient size of about 900 bp for PCR amplification. After the digest the DNA was purified with QIAquick-spin PCR Purification Kit (available from Quiagen, Hilden, Germany).

### 2. Ligation of adapters to fragmented genomic DNA

200 ng of the Csp6I digested and purified DNA were used in the ligation to 50 pmol APL1632 linker. APL1632 is a partial annealing product of 2 oligos: APL16 (SEQ NO:4) and LR32 (SEQ NO:5). Annealing of these 2 oligos results in a TA overhang that can be ligated to the Csp6I sticky end. The ligation was carrierd out with the T4 DNA Ligase and the ligation buffer from Promega Serva, Heidelberg, Germany, at 16 °C over night. After ligation the purification was repeated with the Quiaquick-spin PCR Purification Kit.

### 3. Biotinylated En primer extension reactions

Primer extension reactions were carried out on the adapter linked genomic DNA fragments using the En specific primer En 205R (SEQ NO:6). This primer was biotinylated in order to select specifically synthesized molecules of that reaction. The reaction mixture was as follows:

| | |
|---|---|
| DNA with linkers (50 ng) | 12.5 µl |
| 10x PCR buffer | 5.0 µl |
| 10x dNTPs (2mM) | 5.0 µl |
| 20x TMAC (1mM) (tetra methyl ammonium chloride) | 2.5 µl |
| biotinylated En 205R | 10 pmol |
| Taq Polymerase (from Boehringer, Mannheim, Germany | 2.5 Units |
| water | to 50 µl |

The reaction covered with mineral oil was done on a thermocycler TRIO-block with the following program (12 cycles):

| | |
|---|---|
| 90 °C | 30 sec |
| 94.5 °C | 35 sec |
| 64 °C | 90 sec |
| 73 °C | 300 sec |
| 4 °C | pause |

The QIAquick-spin purification was repeated and the DNA eluted with 50 µl 10 mM Tris/HCl, pH 8.0.

### 4. Specific binding to Dynabeads M-280 streptavidine coated beads

To get rid of the non template DNA M-280 streptavidine coated beads (Dynal, Oslo, Norway) were used. 50 µl 2M NaCl were added to the purified DNA solution (50 µl). The Dynabeads were washed and resuspended in the original volume of B&W buffer (2 mM NaCl/TE, pH 7.6) and 10 µl Dynabeads solution were added to 100 µl of DNA solution. The biotinylated En 205R primer specific DNA molecules were bound to Dynabeads M-280 streptavidine coated beads during incubation for 30 minutes at room temperature. The beads were washed using the Dynal MPC magnet as follows:
2 x 200 µl B&W buffer
2 x 200 µl TE, pH 7.6 / 0.01 % Tween 20.
Then the beads were resuspended in 200 µl TE, pH 7.6 / 0.01 % Tween 20 and stored at 4 °C.

### 5. PCR preamplification

40 µl of the DNA-Dynabeads complex with the buffer removed on the magnet were taken for the following PCR reaction (25 µl):

| | |
|---|---|
| 10x buffer | 2.5 µl |
| 10x dNTPs (2 mM) | 2.5 µl |
| 20x TMAC (1 mM) | 1.25 µl |
| LR26 linker primer (SEQ:NO 7) | 25 pmol |
| En 91R (SEQ:NO 8) | 25 pmol |
| Tag polymerase (Boehringer) | 0.25 Units |
| water | to 25 µl |

The following PCR program (30 cycles) was run:

| | |
|---|---|
| 82 °C | pause |
| 94 °C | 35 sec |
| 64 °C | 60 sec |
| 73 °C | 90 sec |
| 73 °C | 180 sec |
| 4 °C | pause |

During the 82 °C pause the Tag polymerase was added under the oil (hot start).

### 6. Primer extensions with radioactively labelled primer

PCR amplified DNA fragments were used as primer extension reactions. En primers used in these reactions were radioactively labeled with gamma-³²P-ATP by T4 polynucleotide kinase (from New England Biolabs, Schwalbach, Germany. For one radioactive primer extension reaction in 10 µl a mixture was made of

| | |
|---|---|
| preamplification reaction | 1.6 µl |
| 10x buffer | 0.84 µl |
| 10x dNTPs | 0.84 µl |
| 20x TMAC | 0.42 µl |
| labelled primer En 48R (SEQ:NO 9) | 0.75 pmol |
| Tag polymerase (Boehringer) | 0.05 Units |
| Water | to 10 µl |

The reaction mix was incubated on a thermocycler running the following program:

| | |
|---|---|
| 82 °C | 60-120 sec |
| 94 °C | 35 sec |
| 64 °C | 60 sec |
| 73 °C | 90 sec |
| 73 °C | 180 sec |
| 4 °C | pause |

25 cycles of primer extension reaction were carried out. 6 µl of DNA sequencing stop solution (Promega) were added afterwards.

### 7. Separation of the radioactively labelled DNA on a polyacrylamide gel and excision of the fragment of interest

2-4 µg of the primer extension products as well as radioactively labelled 10 bp DNA ladder (Sequamark, Research Genetics, Inc., Huntsville, AL, USA) were denatured and loaded on a 6 % polyacrylamide gel electrophoresis (PAGE). After separation of the DNA the gel was exposed to a X-ray (without fixation) to detect the radioactive bands and their molecular size. The radioactive band of the flanking DNA of the pin1 typic En insertion was detected by its predicted molecular size. The gel fragment containing this DNA of interest was cut out and incubated in 50 µl TE, pH 8.0, to make the DNA diffuse out of the gel slice. The DNA eluted from the gel was PCR amplified using the En specific primer En 48R (SEQ:NO 9) and the adapter specific primer LR26 (SEQ:NO 7). In order to make sure that really flanking genomic DNA of an En element was islated and to obtain its DNA sequence cycling sequencing was carrierd out with the excised and PCR amplified DNA. Cycling sequencing was carried out with the Promega fmol DNA Sequencing System using a gamma-³²P-ATP end-labelled En 48R (SEQ:NO 9) primer. The obtained DNA sequence showed the transition of the En left end sequence to genomic DNA proving that En flanking DNA had been isolated. To show that the flanking DNA of the pin1 typic En element has been isolated the prepared Southern blots were hybridized with a flanking DNA probe synthesized by PCR on the isolated DNA using the primers En 1R (SEQ:NO 10) and LR26 (SEQ:NO 7).

### EXAMPLE IV

### Cloning the complete pin1 cDNA

To get the full size cDNA of the pin1 gene a lambda phage cDNA library of an Arabidopsis thaliana suspension culture was screened. The cDNA library was prepared in the Uni-ZAP XR vector of the lambda ZAPII system from Stratagene, La Jolla, CA, USA and Heidelberg, Germany). The radioactively labelled flanking DNA that had been PCR amplified using the primers En 1R (SEQ:NO 10) and LR 26 (SEQ:NO 7) served as probe for the phage screening. In the second plating positive signals could be detected from single phage plaques. The pBluescript phagemid carrying the pin1 cDNA was obtained from the positive lambda phages in Escherichia coli using the in vivo excision protocol of the ZAP-cDNA Gigapack II Gold Cloning Kit (Stratagene). The excised pBluescript vectors carrying pin1 cDNAs (pcpin1) were checked on a Southern blot with the same probe that was used for the isolation of the single lambda phages.

### EXAMPLE V

### Sequence analysis of the pin1 cDNA clones

### 1. Base pair sequence of the cDNA clone pcpin1/23

The DNA sequence of the cDNA clone pcpin1/23 (SEQ:NO 11) was obtained by the alignment of sequences from deletion clones of pcpin1/23. The deletion clones produced by exonuclease III (New England Biolabs, Schwalbach, Germany) reactions were sequenced on the automated ALF sequencer from Pharmacia. Mapping the isolated flanking DNA of the pin1 typic En insertion on the sequence of the pin1 cDNA shows the insertion site of the En transposon on the level of the cDNA. Figure 2 depicts the insertion of the En element in the pin1 cDNA.

### 2. Open reading frame

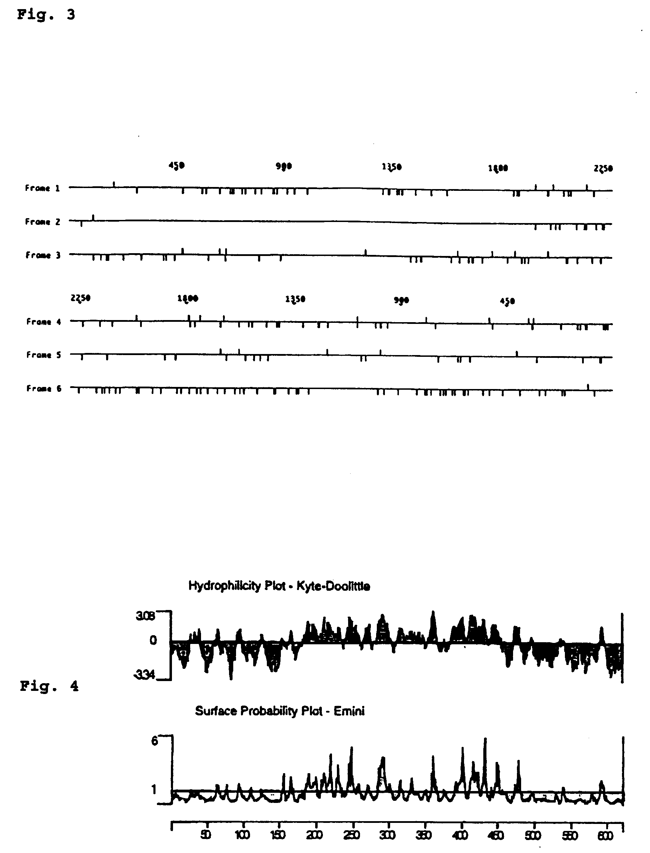

The open reading frame given by DNA computer programs (GCG, University of Wisconsin Computer group, USA; DNA Star) is shown in Figure 3. The most obvious open reading frame is starting from an ATG at position 101 extending to the stop codon TGA at position 1966.

### 3. Predicted characteristics of the pin1 protein

The hydrophilic and hydrophobic characteristics of a protein encoded by the open reading frame of the pin1 cDNA and the surface probability are shown in Figure 4. The plots are made by DNAstar, a computer DNA analysing program using the Kyte-Doolittle and the Emini algorythms. The translated open reading frame shows a protein of 622 amino acids, that has a quite hydrophilic middle part, whereas the 5' and 3' ends consist of several hydrophobic domains. The surface probability follows that triple division of the protein.

### Example VI

### Mapping the genomic position of the pin1 gene

### 1. Mapping on the CIC YAC library

The CIC library is a genomic library of Arabidopsis thaliana (ecotype Colombia) in Yeast Artificial Chromosomes (YAC). It consists of 1152 clones with an average insert size of 420 kb. The library represents around 4 nuclear genome equivalents (Creusot et al. (1995), The Plant J. 8, 763-770). The nitrocellulose filters on which the CIC library YAC clone DNA were arrayed were hybridized with the radioactively labelled full size pin1 cDNA. The autoradiogram showed besides several clones with fainter signals four YAC clones with an equally intensive signal. The YAC clone numbers are the following:
CIC6H1
CIC9C4
CIC12H9
CIC2F9.
On the physical genome map of Arabidopsis thaliana all four clones could be localized close together at the bottom of chromosome 1 beween the markers nga111 and m532. Bennett et al. (1995), The Plant J. 8, 505-520, mapped the pin-formed locus (pin1 gene) in the same chromosomal region on the genetic map of Arabidopsis thaliana close to the crabs claw locus.

### 2. Allelic test

In order to check whether the En pin1 mutation in Arabidopsis thaliana is allelic to the pin-formed 1-1 mutation (Okada et al., supra), both mutants heterozygous for the pin1 mutation were crossed. The F1 generation showed pin1 phenotypes and wild type phenotypes in a ratio that approximated 1:3, what is expected for a Mendelian segragation of two recessive allels in a crossing. Therefore the En pin1 mutation and the pin-formed 1-1 mutations (Okada et al., supra) are allelic.

The cDNA of the pin2 gene from A. thaliana (SEQ:NO 12, pcpin2/4) was obtained by the same procedure as described above for the cDNA of the pin1 gene.

## Claims

1. DNA sequences that code for a pin protein or an active fragment thereof, and the alleles as well as derivatives of these DNA sequences.

2. DNA sequences according to claim 1 isolated from plants.

3. DNA sequences according to claim 1 or 2 wherein said protein is involved in polar auxin transport and/or auxin activity.

4. DNA sequences according to one of the preceding claims wherein said protein is essential for polar auxin transport activity and/or auxin activity.

5. DNA sequences according to one of the preceding claims wherein said protein is located to the plasma membrane and/or associated with the cytoskeleton.

6. DNA sequences according to one of the preceding claims wherein said protein is an auxin receptor protein and/or an auxin carrier protein and/or in contact with an auxin receptor protein and/or an auxin carrier protein.

7. DNA sequences according to one of the preceding claims wherein said protein controls the activity of the auxin efflux carrier proteins or of other proteins involved in polar auxin transport.

8. DNA sequences according to one of the preceding claims wherein said protein is involved in the control of apical dominance, differentiation of the vascular tissues, branching, plant growth, morphogenesis, plant habit, determination and maintenance of tissue polarity, fruit ripening, and/or abscission.

9. DNA sequences according to one of the preceding claims derived from Arabidopsis thaliana.

10. DNA sequences according to one of the preceding claims that code for the pin1 gene product or an active fragment thereof.

11. DNA sequences according to one of the claims 1 to 9 that code for the pin2 gene product or an active fragment thereof.

12. SEQ:NO 11 (cDNA clone pcpin1/23 of the pin1 gene):

1 GTCTCGAGTT TTTTTTTCTT TTTTCCCTCT CACCACTTCT CTCTCAAACT
51 AAAGACAAAA GCTCTTCTCT CTTCCCTCTC TCTTCTCCGG CGAACAAAAA
101 GATGATTACG GCGGCGGACT TCTACCACGT TATGACGGCT ATGGTTCCGT
151 TATACGTAGC TATGATCCTC GCTTACGGCT CTGTCAAATG GTGGAAAATC
201 TTCACACCAG ACCAATGCTC CGGCATAAAC CGTTTCGTCG CTCTCTTCGC
251 CGTTCCTCTC CTCTCTTTCC ACTTCATCGC CGCTAACAAC CCTTACGCCA
301 TGAACCTCCG TTTCCTCGCC GCAGATTCTC TCCAGAAAGT CATTGTCCTC
351 TCTCTCCTCT TCCTCTGGTG CAAACTCAGC CGCAACGGTT CTTTAGATTG
401 GACCATAACT CTCTTCTCTC TCTCGACACT CCCCAACACT CTAGTCATGG
451 GGATACCTCT TCTCAAAGGC ATGTATGGTA ATTTCTCCGG CGACCTCATG
501 GTTCAAATCG TTGTTCTTCA GTGTATCATT TGGTACATAC TCATGCTCTT
551 TCTCTTTGAG TACCGTGGAG CTAAGCTTTT GATCTCCGAG CAGTTTCCAG
601 ACACAGCAGG ATCTATTGTT TCGATTCATG TTGATTCCGA CATTATGTCT
651 TTAGATGGAA GACAACCTTT GGAAACTGAA GCTGAGATTA AAGAAGATGG
701 GAAGCTTCAT GTTACTGTTC GTCGTTCTAA TGCTTCAAGG TCTGATATTT
751 ACTCGAGAAG GTCTCAAGGC TTATCTGCGA CACCTAGACC TTCGAATCTA
801 ACCAACGCTG AGATATATTC GCTTCAGAGT TCAAGAAACC CAACGCCACG
851 TGGCTCTAGT TTTAATCATA CTGATTTTTA CTCGATGATG GCTTCTGGTG
901 GTGGTCGGAA CTCTAACTTT GGTCCTGGAG AAGCTGTGTT TGGTTCTAAA
951 GGTCCTACTC CGAGACCTTC CAACTACGAA GAAGACGGTG GTCCTGCTAA
1001 ACCGACGGCT GCTGGAACTG CTGCTGGAGC TGGGAGGTTT CATTATCAAT
1051 CTGGAGGAAG TGGTGGCGGT GGAGGAGCGC ATTATCCGGC GCCGAACCCA
1101 GGGATGTTTT CGCCCAACAC TGGCGGTGGT GGAGGCACGG CGGCGAAAGG
1151 AAACGCTCCG GTGGTTGGTG GGAAAAGACA AGACGGAAAC GGAAGAGATC
1201 TTCACATGTT TGTGTGGAGC TCAAGTGCTT CGCCGGTCTC AGATGTGTTC
1251 GGCGGTGGAG GAGGAAACCA CCACGCCGAT TACTCCACCG CTACGAACGA
1301 TCATCAAAAG GACGTTAAGA TCTCTGTACC TCAGGGGAAT AGTAACGACA
1351 ACCAGTACGT GGAGAGGGAA GAGTTTAGTT TCGGTAACAA AGACGATGAT

1401 AGCAAAGTAT TGGCAACGGA CGGTGGGAAC AACATAAGCA ACAAAACGAC
1451 GCAGGCTAAG GTGATGCCAC CAACAAGTGT GATGACAAGA CTCATTCTCA
1501 TTATGGTTTG GAGGAAACTT ATTCGTAATC CCAACTCTTA CTCCAGTTTA
1551 TTCGGCATCA CCTGGTCCCT CATTTCCTTC AAGTGGAACA TTGAAATGCC
1601 AGCTCTTATA GCAAAGTCTA TCTCCATACT CTCAGATGCA GGTCTAGGCA
1651 TGGCTATGTT CAGTCTTGGG TTGTTCATGG CGTTAAACCC AAGAATAATA
1701 GCTTGTGGAA ACAGAAGAGC AGCTTTTGCG GCGGCTATGA GATTTGTCGT
1751 TGGACCTGCC GTCATGCTCG TTGCTTCTTA TGCCGTTGGC CTCCGTGGCG
1801 TCCTCCTCCA TGTTGCCATT ATCCAGGCAG CTTTGCCGCA AGGAATAGTA
1851 CCGTTTGTGT TTGCCAAAGA GTATAATGTG CATCCTGACA TTCTTAGCAC
1901 TGCGGTGATA TTTGGGATGT TGATCGCGTT GCCCATAACT CTTCTCTACT
1951 ACATTCTCTT GGGTCTATGA AGAGATATTA CCAAAACACA GGGACTTTGT
2001 TTTATTCTTT TGTGGGATGA TGAATTGTGA AAAGAACAAT GCCCTTTTTG
2051 TTGAAAACCC ACAAATTAAA TCAGAAGCAG CTTTAGAGAA TCTTTGAGGA
2101 TAATTGAAGC TCTTGAAGAA GAGAAGAAGA AGGAGACTTA AGTAGGAGCT
2151 CAGCAAGTTT TACCTTTTTC TTAATTTTAA TGAACATTCG TGTTTCCTCT
2201 TTTGGTAGGT TTTAGGAATT TGTAAAAGCT TTGGCTACTT TTAGTGAATT
2251 AAAAACGTTA AGGAAAATAT CAAAAAAAAA AAAAAAAAAA AA

13. SEQ:NO 12 (cDNA clone pcpin2/4 of the pin2 gene):

1   GGCACGAGCC GGTTCGTTGC GGTTTTCGCG GTTCCTCTTC TCTCTTTCCA
51  TTTCATCTCC TCCAATGATC CTTATGCAAT GAATTACCAC TTCCTCGCTG
101 CTGATTCTCT TCAGAAAGTC GTTATCCTCG CCGCACTCTT TCTTTGGCAG
151 GCGTTTAGCC GCAGAGGAAG CCTAGAATGG ATGATAACGC TCTTTTCACT
201 ATCAACACTG CCTAACACGT TGGTAATGGG AATCCCATTG CTTAGGGCGA
251 TGTACGGAGA CTTCTCCGGT AACCTAATGG TGCAGATCGT GGTGCTTCAG
301 AGCATCATAT GGTATACATT AATGCTCTTC TTGTTTGAGT TCCGTGGGGC
351 TAAGCTTCTC ATCTCCGAGC AGTTCCCGGA GACGGCTGGT TCAATTACTT
401 CCTTCAGAGT TGACTCTGAT GTTATCTCTC TTAATGGCCG TGAACCCCTC
451 CAGACCGATG CGGAGATAGG AGACGACGGA AAGCTACACG TGGTGGTTCG
501 AAGATCAAGT GCCGCCTCAT CAATGATCTC TTCATTCAAC AAATCTCACG
551 GCGGAGGACT TAACTCCTCC ATGATAACGC CGCGAGCTTC AAATCTCACC
601 GGCGTAGAGA TTTACTCCGT TCAATCGTCA CGAGAGCCGA CGCCGAGAGC
651 TTCTAGCTTT AATCAGACAA ATTTCTACGC AATGTTTAAC GCAAGCAAAG
701 CTCCAAGCCC TCGTCACGGT TACACTAATA GCTACGGCGG CGCTGGAGCT

751 GGTCCAGGTG GAGATGTTTA CTCACTTCAG TCTTCTAAAG GCGTGACGCC
801 GAGAACGTCA AATTTTGATG AGGAAGTTAT GAAGACGGCG AAGAAAGCAG
851 GAAGAGGAGG CAGAAGTATG AGTGGGGAAT TATACAACAA TAATAGTGTT
901 CCGTCGTACC CACCGCCGAA CCCAATGTTC ACGGGGTCAA CGAGTGGAGC
951 AAGTGGAGTC AAGAAAAAGG AAAGTGGTGG CGGAGGAAGC GGTGGCGGAG
1001 TAGGAGTAGG AGGACAAAAC AAGGAGATGA ACATGTTCGT GTGGAGTTCG
1051 AGTGCTTCTC CGGTGTCGGA AGCCAACGCG AAGAATGCTA TGACCAGAGG
1101 TTCTTCCACC GATGTATCCA CCGACCCTAA AGTTTCTATT CCTCCTCACG
1151 ACAACCTCGC TACTAAAGCG ATGCAGAATC TGATAGAGAA CATGTCACCG
1201 GGAAGAAAAG GGCATGTGGA AATGGACCAA GACGGTAATA ACGGGGGAAA
1251 GTCACCTTAC ATGGGCAAAA AAGGTAGCGA CGTGGAAGAC GGCGGTCCCG
1301 GTCCTAGGAA ACAGCAGATG CCGCCGGCGA GTGTGATGAC GAGACTAATT
1351 CTGATAATGG TTTGGAGAAA ACTCATTCGA AACCCTAACA CTTACTCTAG
1401 TCTCTTTGGC CTTGCTTGGT CCCTTGTCTC TATCAAGTGG AATATAAAGA
1451 TGCCAACGAT AATGAGTGGA CCGATTTCGA TATTATCTGA TGCTGGTCTT
1501 GGAATGGCTA TGTTTAGTCT TGGTCTATTT ATGGCATTGC AACCAAAGAT
1551 TATTGCGTGC GGAAAATCAG TAGCAGGGTT TGCGATGGCC GTAAGGTTCT
1601 TGACTGGACC AGCCGTGATC GCAGCCACCT CAATAGCAAT TGGTATTCGA
1651 GGTGATCTCC TCCATATCGC CATCGTTCAG GCTGCTCTTC CTCAAGGAAT
1701 CGTTCCTTTT GTTTTCGCCA AAGAATATAA CGTCCATCCT GATATTCTCA
1751 GCACTGCGGT TATATTCGGA ATGCTGGTTG CTTTGCCTGT AACAGTACTC
1801 TACTACGTTC TTTTGGGGCT TTAAGTTATT ATCAAAACGT ATTTGCAAAT
1851 AAAAGGCGAT ACGACCCAAA GGTGATTTTT TTTCAAACGA AAAAGAATAA
1901 TTACAAGAAC GAAAAAAGAC TAATTCCAGG TCAGAGGCTT AGGTGTATGG
1951 GACCATGCAA TGTCGCATTA ATTAAATTAT AGCATATGAT AGTCGAAAAT
2001 TTAGATAACT TTGTATAATT AATTATATGC ACATGCATGT ACGTGACTTT
2051 GTAGTTTTTG TTACATTTAT TAAATTTTTG GGATGTGCAA GTACAATTAT
2101 TACTAAAAAA AAAAAAA

14. Proteins or fragments thereof encoded by the DNA sequences according to one of the preceding claims.

15. Proteins or fragments thereof encoded by the pin1 gene.

16. Proteins or fragments thereof encoded by the pin2 gene.

17. Protein encoded by the DNA sequence according to claim 12 or fragments thereof.

18. Protein encoded by the DNA sequence according to claim 13 or fragments thereof.

19. Proteins or fragments thereof according to one of claims 14-18 from Arabidopsis thaliana.

20. Nucleic acid molecules comprising the coding region of the pin1 gene, the pin2 gene or fragments thereof.

21. Nucleic acid molecules according to claim 20 wherein said coding region is combined with regulatory sequences.

22. Nucleic acid molecules according to claim 20 or 21 wherein said regulatory sequences control tissue-specific, developmental specific and/or constitutive gene expression.

23. Nucleic acid molecules according one of claims 20 to 22 wherein said regulatory sequences control inducible gene expression and the induction stimulus can be abiotic or biotic.

24. Nucleic acid molecules according to one of claims 20 to 23 comprising additional enhancer, transposon and/or other regulatory sequences.

25. Nucleic acid molecules according to one of claims 20 to 24 wherein said coding region or fragments thereof are in the antisense orientation.

26. Vectors comprising a DNA sequence according to claims 1 to 13 or a nucleic acid molecule according to claims 20 to 25.

27. Microorganism or animal cells containing a DNA sequence according to one of the claims 1 to 13 or 20 to 26.

28. E. coli clone DSM 10712 (containing pcpin1/23).

29. Plants containing at least one of the DNA sequences according to one of claims 1 to 13, at least one of the nucleic acid molecules according to one of claims 20 to 26, or at least one of the microorganisms according to claims 27 or 28, as well as parts and products of these plants, including protoplasts, plant cells, calli, seeds, tubers, cuttings, etc., as well as the progenies of these plants.

30. Plants according to claim 29 wherein polar auxin transport or auxin activity is altered in said plants.

31. Plants according to claim 29 or 30 wherein plant growth, architecture, branching, morphogenesis, differentiation of the vascular tissues, abscission, flowering, fruit ripening, and/or other auxin controlled developmental processes are altered.

32. Dicotyledons according to one of claims 29 to 31.

33. Monocotyledons according to one of claims 29 to 31.

34. Plants according to claim 33 wherein said plant is wheat, maize, rye, oats, millet, barley, rice, maize.

35. Gymnosperms according to one of claims 29 to 31.

36. Plants according to claim 35 wherein said plant is poplar, picea or eucalyptus.

37. Method for producing plants, plant parts, plant products according to one of claims 29 to 36.

38. Use of DNA sequences and/or nucleic acid molecules according to one of claims 1 to 13 or 20 to 26 for the isolation, and use of fragments thereof or of the gene product or fragments thereof for the isolation of DNA sequences that code for proteins involved in polar auxin transport.

39. Use of the proteins according to one of claims 14 to 19 and the use of fragments thereof for the isolation of proteins involved in polar auxin transport.

40. Use according to claim 38 or 39 for the isolation of pin genes, fragments thereof or homologous DNA sequences or of pin proteins.

41. Use of DNA sequences and/or nucleic acid molecules according to one of claims 1 to 13 or 20 to 26 for producing plants which have an altered polar auxin transport or auxin activity.

42. Use according to claim 37 for producing plants in which plant growth, wood production, architecture, branching, morphogenesis, differentiation of the vascular tissues, abscission, flowering, fruit ripening, root formation and/or other auxin controlled developmental processes are altered.


SEQUENCE LISTING

(1) GENERAL INFORMATION:

(i) APPLICANT:
(A) NAME: Max-Planck-Gesellschaft zur Foerderung der
Wissenschaften e.V.
(B) STREET: Hofgartenstrasse 2
(C) CITY: Muenchen
(E) COUNTRY: Germany
(F) POSTAL CODE (ZIP): 80539

(ii) TITLE OF INVENTION: Genetic control of polar auxin transport in
plants and manipulation of plant growth, architecture and
morphogenesis

(iii) NUMBER OF SEQUENCES: 12

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "oligonucleotide"

(xi) SEQUENCE DESCRIPTION: SEQ ID NO: 1:

GCAGATGTAT GGAGGTGCCG GAAC 24

(2) INFORMATION FOR SEQ ID NO: 2:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "oligonucleotide"

(xi) SEQUENCE DESCRIPTION: SEQ ID NO: 2:

CGAGTGTAGT ACGATCAGGC GGAG 24

(2) INFORMATION FOR SEQ ID NO: 3:

(i) SEQUENCE CHARACTERISTICS:

(A) LENGTH: 24 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "oligonucleotide"

(xi) SEQUENCE DESCRIPTION: SEQ ID NO: 3:

AGGTTGGGCA GAAACATTTT GCTT 24

(2) INFORMATION FOR SEQ ID NO: 4:

(i) SEQUENCE CHARACTERISTICS:
(A) LENGTH: 16 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "oligonucleotide"

(xi) SEQUENCE DESCRIPTION: SEQ ID NO: 4:

TATGGGATCA CATTAA 16

(2) INFORMATION FOR SEQ ID NO: 5:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "oligonucleotide"

(xi) SEQUENCE DESCRIPTION: SEQ ID NO: 5:

ACTCGATTCT CAACCCGAAA GTATAGATCC CA 32

(2) INFORMATION FOR SEQ ID NO: 6:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "oligonucleotide"

(xi) SEQUENCE DESCRIPTION: SEQ ID NO: 6:

AGAAGCACGA CGGCTGTAGA ATAGGA 26

(2) INFORMATION FOR SEQ ID NO: 7:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "oligonucleotide"

(xi) SEQUENCE DESCRIPTION: SEQ ID NO: 7:

ACTCGATTCT CAACCCGAAA GTATAG 26

(2) INFORMATION FOR SEQ ID NO: 8:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "oligonucleotide"

(xi) SEQUENCE DESCRIPTION: SEQ ID NO: 8:

TGCAGCAAAA CCCACACTTT TACTTC 26

(2) INFORMATION FOR SEQ ID NO: 9:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "oligonucleotide"

(xi) SEQUENCE DESCRIPTION: SEQ ID NO: 9:

TTTACTTCGA TTAAGAGTGT CGGCC 25

(2) INFORMATION FOR SEQ ID NO: 10:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "oligonucleotide"

(xi) SEQUENCE DESCRIPTION: SEQ ID NO: 10:

ACTCCTTTGA CGTTTTCTTG TAGTG 25

(2) INFORMATION FOR SEQ ID NO: 11:

(i) SEQUENCE CHARACTERISTICS:
(A) LENGTH: 2292 base pairs
(B) TYPE: nucleic acid
(C) STRANDEDNESS: double
(D) TOPOLOGY: linear

(ii) MOLECULE TYPE: cDNA to mRNA

(xi) SEQUENCE DESCRIPTION: SEQ ID NO: 11:

GTCTCGAGTT TTTTTTTCTT TTTTCCCTCT CACCACTTCT CTCTCAAACT AAAGACAAAA 60

GCTCTTCTCT CTTCCCTCTC TCTTCTCCGG CGAACAAAAA GATGATTACG GCGGCGGACT 120

TCTACCACGT TATGACGGCT ATGGTTCCGT TATACGTAGC TATGATCCTC GCTTACGGCT 180

CTGTCAAATG GTGGAAAATC TTCACACCAG ACCAATGCTC CGGCATAAAC CGTTTCGTCG 240

CTCTCTTCGC CGTTCCTCTC CTCTCTTTCC ACTTCATCGC CGCTAACAAC CCTTACGCCA 300

TGAACCTCCG TTTCCTCGCC GCAGATTCTC TCCAGAAAGT CATTGTCCTC TCTCTCCTCT 360

TCCTCTGGTG CAAACTCAGC CGCAACGGTT CTTTAGATTG GACCATAACT CTCTTCTCTC 420

TCTCGACACT CCCCAACACT CTAGTCATGG GGATACCTCT TCTCAAAGGC ATGTATGGTA 480

ATTTCTCCGG CGACCTCATG GTTCAAATCG TTGTTCTTCA GTGTATCATT TGGTACATAC 540

TCATGCTCTT TCTCTTTGAG TACCGTGGAG CTAAGCTTTT GATCTCCGAG CAGTTTCCAG 600

ACACAGCAGG ATCTATTGTT TCGATTCATG TTGATTCCGA CATTATGTCT TTAGATGGAA 660

GACAACCTTT GGAAACTGAA GCTGAGATTA AAGAAGATGG GAAGCTTCAT GTTACTGTTC 720

GTCGTTCTAA TGCTTCAAGG TCTGATATTT ACTCGAGAAG GTCTCAAGGC TTATCTGCGA 780

CACCTAGACC TTCGAATCTA ACCAACGCTG AGATATATTC GCTTCAGAGT TCAAGAAACC 840

CAACGCCACG TGGCTCTAGT TTTAATCATA CTGATTTTTA CTCGATGATG GCTTCTGGTG 900

GTGGTCGGAA CTCTAACTTT GGTCCTGGAG AAGCTGTGTT TGGTTCTAAA GGTCCTACTC 960

CGAGACCTTC CAACTACGAA GAAGACGGTG GTCCTGCTAA ACCGACGGCT GCTGGAACTG 1020

CTGCTGGAGC TGGGAGGTTT CATTATCAAT CTGGAGGAAG TGGTGGCGGT GGAGGAGCGC 1080

ATTATCCGGC GCCGAACCCA GGGATGTTTT CGCCCAACAC TGGCGGTGGT GGAGGCACGG 1140

CGGCGAAAGG AAACGCTCCG GTGGTTGGTG GGAAAAGACA AGACGGAAAC GGAAGAGATC 1200

TTCACATGTT TGTGTGGAGC TCAAGTGCTT CGCCGGTCTC AGATGTGTTC GGCGGTGGAG 1260

GAGGAAACCA CCACGCCGAT TACTCCACCG CTACGAACGA TCATCAAAAG GACGTTAAGA 1320

TCTCTGTACC TCAGGGGAAT AGTAACGACA ACCAGTACGT GGAGAGGGAA GAGTTTAGTT 1380

TCGGTAACAA AGACGATGAT AGCAAAGTAT TGGCAACGGA CGGTGGGAAC AACATAAGCA 1440

ACAAAACGAC GCAGGCTAAG GTGATGCCAC CAACAAGTGT GATGACAAGA CTCATTCTCA 1500

TTATGGTTTG GAGGAAACTT ATTCGTAATC CCAACTCTTA CTCCAGTTTA TTCGGCATCA 1560

CCTGGTCCCT CATTTCCTTC AAGTGGAACA TTGAAATGCC AGCTCTTATA GCAAAGTCTA 1620

TCTCCATACT CTCAGATGCA GGTCTAGGCA TGGCTATGTT CAGTCTTGGG TTGTTCATGG 1680

CGTTAAACCC AAGAATAATA GCTTGTGGAA ACAGAAGAGC AGCTTTTGCG GCGGCTATGA 1740

GATTTGTCGT TGGACCTGCC GTCATGCTCG TTGCTTCTTA TGCCGTTGGC CTCCGTGGCG 1800

TCCTCCTCCA TGTTGCCATT ATCCAGGCAG CTTTGCCGCA AGGAATAGTA CCGTTTGTGT 1860

TTGCCAAAGA GTATAATGTG CATCCTGACA TTCTTAGCAC TGCGGTGATA TTTGGGATGT 1920

TGATCGCGTT GCCCATAACT CTTCTCTACT ACATTCTCTT GGGTCTATGA AGAGATATTA 1980

CCAAAACACA GGGACTTTGT TTTATTCTTT TGTGGGATGA TGAATTGTGA AAAGAACAAT 2040

GCCCTTTTTG TTGAAAACCC ACAAATTAAA TCAGAAGCAG CTTTAGAGAA TCTTTGAGGA 2100

TAATTGAAGC TCTTGAAGAA GAGAAGAAGA AGGAGACTTA AGTAGGAGCT CAGCAAGTTT 2160

TACCTTTTTC TTAATTTTAA TGAACATTCG TGTTTCCTCT TTTGGTAGGT TTTAGGAATT 2220

TGTAAAAGCT TTGGCTACTT TTAGTGAATT AAAAACGTTA AGGAAAATAT CAAAAAAAAA 2280

AAAAAAAAAA AA 2292

(2) INFORMATION FOR SEQ ID NO: 12:

(i) SEQUENCE CHARACTERISTICS:
(A) LENGTH: 2117 base pairs
(B) TYPE: nucleic acid
(C) STRANDEDNESS: double
(D) TOPOLOGY: linear

(ii) MOLECULE TYPE: cDNA to mRNA

(xi) SEQUENCE DESCRIPTION: SEQ ID NO: 12:

GGCACGAGCC GGTTCGTTGC GGTTTTCGCG GTTCCTCTTC TCTCTTTCCA TTTCATCTCC 60

TCCAATGATC CTTATGCAAT GAATTACCAC TTCCTCGCTG CTGATTCTCT TCAGAAAGTC 120

GTTATCCTCG CCGCACTCTT TCTTTGGCAG GCGTTTAGCC GCAGAGGAAG CCTAGAATGG 180

ATGATAACGC TCTTTTCACT ATCAACACTG CCTAACACGT TGGTAATGGG AATCCCATTG 240

CTTAGGGCGA TGTACGGAGA CTTCTCCGGT AACCTAATGG TGCAGATCGT GGTGCTTCAG 300

AGCATCATAT GGTATACATT AATGCTCTTC TTGTTTGAGT TCCGTGGGGC TAAGCTTCTC 360

ATCTCCGAGC AGTTCCCGGA GACGGCTGGT TCAATTACTT CCTTCAGAGT TGACTCTGAT 420

GTTATCTCTC TTAATGGCCG TGAACCCCTC CAGACCGATG CGGAGATAGG AGACGACGGA 480

AAGCTACACG TGGTGGTTCG AAGATCAAGT GCCGCCTCAT CAATGATCTC TTCATTCAAC 540

AAATCTCACG GCGGAGGACT TAACTCCTCC ATGATAACGC CGCGAGCTTC AAATCTCACC 600

GGCGTAGAGA TTTACTCCGT TCAATCGTCA CGAGAGCCGA CGCCGAGAGC TTCTAGCTTT 660

AATCAGACAA ATTTCTACGC AATGTTTAAC GCAAGCAAAG CTCCAAGCCC TCGTCACGGT 720

TACACTAATA GCTACGGCGG CGCTGGAGCT GGTCCAGGTG GAGATGTTTA CTCACTTCAG 780

TCTTCTAAAG GCGTGACGCC GAGAACGTCA AATTTTGATG AGGAAGTTAT GAAGACGGCG 840

AAGAAAGCAG GAAGAGGAGG CAGAAGTATG AGTGGGGAAT TATACAACAA TAATAGTGTT 900

CCGTCGTACC CACCGCCGAA CCCAATGTTC ACGGGGTCAA CGAGTGGAGC AAGTGGAGTC 960

AAGAAAAAGG AAAGTGGTGG CGGAGGAAGC GGTGGCGGAG TAGGAGTAGG AGGACAAAAC 1020

AAGGAGATGA ACATGTTCGT GTGGAGTTCG AGTGCTTCTC CGGTGTCGGA AGCCAACGCG 1080

AAGAATGCTA TGACCAGAGG TTCTTCCACC GATGTATCCA CCGACCCTAA AGTTTCTATT 1140

CCTCCTCACG ACAACCTCGC TACTAAAGCG ATGCAGAATC TGATAGAGAA CATGTCACCG 1200

GGAAGAAAAG GGCATGTGGA AATGGACCAA GACGGTAATA ACGGGGGAAA GTCACCTTAC 1260

ATGGGCAAAA AAGGTAGCGA CGTGGAAGAC GGCGGTCCCG GTCCTAGGAA ACAGCAGATG 1320

CCGCCGGCGA GTGTGATGAC GAGACTAATT CTGATAATGG TTTGGAGAAA ACTCATTCGA 1380

AACCCTAACA CTTACTCTAG TCTCTTTGGC CTTGCTTGGT CCCTTGTCTC TATCAAGTGG 1440

AATATAAAGA TGCCAACGAT AATGAGTGGA CCGATTTCGA TATTATCTGA TGCTGGTCTT 1500

GGAATGGCTA TGTTTAGTCT TGGTCTATTT ATGGCATTGC AACCAAAGAT TATTGCGTGC 1560

GGAAAATCAG TAGCAGGGTT TGCGATGGCC GTAAGGTTCT TGACTGGACC AGCCGTGATC 1620

GCAGCCACCT CAATAGCAAT TGGTATTCGA GGTGATCTCC TCCATATCGC CATCGTTCAG 1680

GCTGCTCTTC CTCAAGGAAT CGTTCCTTTT GTTTTCGCCA AAGAATATAA CGTCCATCCT 1740

GATATTCTCA GCACTGCGGT TATATTCGGA ATGCTGGTTG CTTTGCCTGT AACAGTACTC 1800

TACTACGTTC TTTTGGGGCT TTAAGTTATT ATCAAAACGT ATTTGCAAAT AAAAGGCGAT 1860

ACGACCCAAA GGTGATTTTT TTTCAAACGA AAAAGAATAA TTACAAGAAC GAAAAAAGAC 1920

TAATTCCAGG TCAGAGGCTT AGGTGTATGG GACCATGCAA TGTCGCATTA ATTAAATTAT 1980

AGCATATGAT AGTCGAAAAT TTAGATAACT TTGTATAATT AATTATATGC ACATGCATGT 2040

ACGTGACTTT GTAGTTTTTG TTACATTTAT TAAATTTTTG GGATGTGCAA GTACAATTAT 2100

TACTAAAAAA AAAAAAA 2117